(19) Europäisches Patentamt
European Patent Office
Office européen des brevets

(11) Publication number: **0 347 925 B1**

(12) **EUROPEAN PATENT SPECIFICATION**

(45) Date of publication of patent specification: **18.11.93**

(51) Int. Cl.5: **C07D 233/90**, A01N 43/50, C07D 249/08, C07D 213/82, C07D 213/83, C07D 239/30, C07D 401/12, A01N 43/653, A01N 43/40, C07D 233/64

(21) Application number: **89111413.4**

(22) Date of filing: **22.06.89**

The file contains technical information submitted after the application was filed and not included in this specification

(54) **Fungicidal heterocyclic nitrogen compounds.**

(30) Priority: **23.06.88 IT 2107788**

(43) Date of publication of application:
**27.12.89 Bulletin 89/52**

(45) Publication of the grant of the patent:
**18.11.93 Bulletin 93/46**

(84) Designated Contracting States:
**AT BE CH DE ES FR GB GR LI NL SE**

(56) References cited:
**EP-A- 0 031 086**
**EP-A- 0 032 199**
**EP-A- 0 121 344**
**EP-A- 0 243 842**
**EP-A- 0 264 577**
**DE-A- 3 629 064**
**DE-A- 3 723 230**
**GB-A- 1 586 998**
**GB-A- 2 187 733**
**US-A- 4 579 856**
**US-A- 4 588 735**

(73) Proprietor: **MINISTERO DELL' UNIVERSITA' E DELLA RICERCA SCIENTIFICA E TECNOLOGICA**
**76, Lungotevere Thaon di Revel**
**I-00196 Roma(IT)**

(72) Inventor: **Camaggi, Giovanni**
**via Ragazzi del 99**
**I-28100 Novara(IT)**
Inventor: **Filippini, Lucio**
**via Fratelli Cervi 27**
**I-21047 Saronno (VA)(IT)**
Inventor: **Garavaglia, Carlo**
**piazza Vittoria 7**
**I-20012 Cuggiono (MI)(IT)**
Inventor: **Mirenna, Luigi**
**via Gamboloita 4**
**I-20139 Milano(IT)**
Inventor: **Venturini, Isabella**
**via Marco d'Agrate 21**
**I-20139 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 347 925 B1

Rank Xerox (UK) Business Services
(3.10/3.6/3.3.1)

(74) Representative: **Weinhold, Peter, Dr. et al**
**Patentanwälte Dr. V. Schmied-Kowarzik**
**Dipl.-Ing. G. Dannenberg**
**Dr. P. Weinhold**
**Dr. D. Gudel**
**Dipl.-Ing. S. Schubert**
**Dr. P.**
**Barz**
**Siegfriedstrasse 8**
**D-80803 München (DE)**

## Description

The present invention relates to heterocyclic nitrogen compounds which are highly fungicidal, to the process for preparing them and to their use as fungicides in the agrarian field.

The EP-A 0 243 842 describes fungicidal compounds of the formula

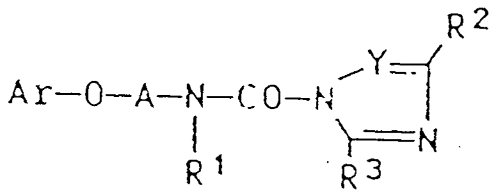

wherein Ar represents phenyl or substituted phenyl, $R^1$ is substituted alkyl, A is a hydrocarbon chain, Y is CH or N and $R^2$ and $R^3$ represent hydrogen or alkyl.

The US-P-4,579,856 discloses fungicidal N-pyridyloxyalkyl amides of formula

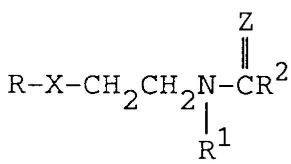

wherein X and Z are oxygen or sulfur; R is an optionally substituted pyridyl; $R^1$ is lower alkyl; and $R^2$ is a 5- or 6-membered heterocyclic ring containing 1 to 3 ring nitrogens.

The GB-A-2,187,733 describes imidazole compounds useful as fungicides having the formula

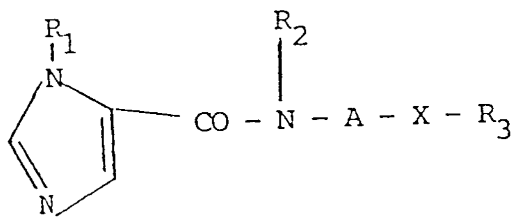

wherein X represents an oxygen or sulphur atom; $R_1$ represents an alkenyl group; $R_2$ represents an optionally substituted alkyl, cycloalkyl, alkenyl or alkynyl group; $R_3$ represents an optionally substituted phenyl group; and A represents an alkylene group.

The US-P-4,588,935 discloses fungicidal (trihalophenoxy or trihalophenthio) alkylaminoalkyl pyridines and pyrroles of the formula

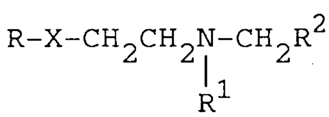

wherein

- X is sulfur or oxygen;
- R is phenyl or phenyl substituted with 1 to 3 substituents independently selected from halogen, lower alkyl or trihalomethyl;
- $R^1$ is lower alkyl or lower alkoxyalkyl; and
- $R^2$ is a 5- or 6-membered heterocyclic ring having 1 to 3 ring nitrogens or the group $-CH_2-R^3$ wherein $R^3$ is a 5- or 6-membered heterocyclic ring having 1 to 3 ring nitrogens.

The problem underlying the present invention is to provide novel fungicidal compounds superior to those of the prior art.

Object of the present invention are therefore compounds of the general formula:

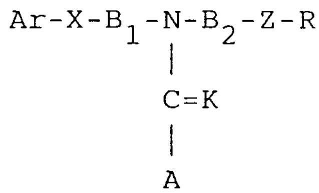

(I)

wherein:

| | |
|---|---|
| Ar | represents phenyl; phenyl substituted with one or more groups selected from halogen atoms, $C_{1-4}$-alkyl $C_{1-4}$-haloalkyl, $C_{2-4}$-alkenyl, $C_{2-4}$-haloalkenyl, $C_{1-3}$-alkoxy and $C_{1-4}$-haloalkoxy groups; pyridyl;and pyridyl substituted with one or more groups selected from halogen atoms and $C_{1-3}$-haloalkyl groups; |
| K, X and Z | are, independently from each other, either O or S; |
| $B_1$ and $B_2$, | the same or different from each other, are linear or branched $C_{1-6}$-alkylene groups; |
| R | represents $C_{1-6}$-haloalkyl containing from 1 to 9 halogen atoms, $C_{2-8}$-haloalkenyl containing from 1 to 9 halogen atoms, $C_{3-8}$-haloalkoxyalkyl, $C_{3-8}$-haloalkoxyalkenyl, with the halogen being preferably fluorine; |
| A | represents a group selected from: |

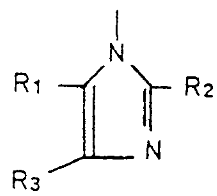
;
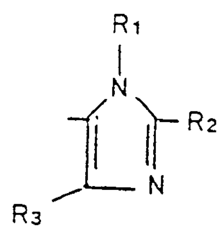
;
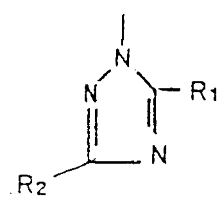
;
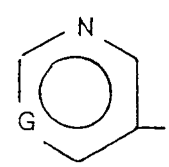

wherein:
$R_1$, $R_2$ and $R_3$ represent H; and
G is either CH or N;
and salts and metal complexes thereof.

Specific examples of the radicals mentioned above are:

| | |
|---|---|
| Halogen: | F, Cl, Br and J, particularly F and Cl; |
| $C_{1-4}$-alkyl: | methyl, ethyl, n-and i-propyl, n, i-, sek.-and tert.-butyl; |
| $C_{1-4}$-haloalkyl: | the above $C_{1-4}$-alkyl groups substituted with 1 up to the maximum number of halogen atoms (the same or different from each other); |
| $C_{2-4}$-alkenyl: | vinyl, 1- and 2-propenyl, butenyl; |
| $C_{2-4}$-haloalkenyl: | the above $C_{2-4}$-alkenyl groups substituted with 1 up to the maximum number of halogen atoms; |
| $C_{1-3}$-alkoxy: | methoxy, ethoxy, n- and i-propoxy; |
| $C_{1-4}$-haloalkoxy: | the above $C_{1-3}$-alkoxy groups and butoxy, each substituted with 1 up to the maximum number of halogen atoms; |
| $C_{1-6}$-alkylene: | methylene, ethylene, n- and ipropylene, butylene, pentylene, hexylene; |
| $C_{1-6}$-haloalkyl: | the above $C_{1-3}$-haloalkyl groups and halobutyl, halopentyl and halohexyl; |

| | |
|---|---|
| $C_{3-8}$-haloalkoxyalkyl: | ethyl, propyl, butyl, pentyl, hexyl and heptyl, each substituted with at least one halogen and at least (and preferably) one (preferably $C_{1-3}$-)alkoxy group; |
| $C_{3-8}$-haloalkoxyalkenyl: | the above $C_{3-8}$-haloalkoxyalkyl groups which contain one carbon-carbon double bond; |
| $C_{1-6}$-haloalkyl: | the above $C_{1-6}$-alkyl groups substituted with 1 up to the maximum number of halogen atoms; |
| $C_{2-6}$-haloalkenyl: | the above $C_{2-4}$-alkenyl groups and pentenyl and hexenyl, substituted with 1 up to the maximum number of halogen atoms; |

Preferred meanings for the above groups are:

| | |
|---|---|
| Ar: | phenyl; phenyl substituted with one to three radicals independently selected from F, Cl, Br, $CF_3$, $C_2F_5$,$OCH_3$, $OC_2F_5$ and $C_{1-4}$-alkyl; |
| K,X and Z: | at least two of them and particularly all of them being oxygen; |
| $B_1$ and $B_2$: | independently ethylene, linear or branched propylene, butylene and hexylene; |
| R: | $C_{1-6}$-haloalkyl containing from 1 to 9 halogen atoms, preferably selected from F and Cl; or $C_{1-3}$-alkyl having 2 up to the maximum number of halogen substituents, independently selected from F and Cl, one substituent optionally being a $C_{1-3}$-alkoxy group, for example $-CF_2CF_2H$, $-CF_2CFHOCF_3$ and $-CF_2CFClH$; |
| A: | 1-imidazolyl, 5-imidazolyl and 3-pyridyl. |

The salts of the compounds of general formula (I) may be derived from an inorganic acid such as a hydrogen halide (for example hydroiodic acid, hydrobromic acid and hydrochloric acid); sulfuric acid, nitric acid, thiocyanic acid and phosphoric acid; or from an organic acid, such as acetic acid, propionic acid, ethanedioic acid, propanedioic acid, benzoic acid, methanesulfonic acid, 4-methylbenzenesulfonic acid, etc.

The metal complexes of the compounds of formula (I) may be obtained by means of a complexation reaction between the compounds of formula (I) and an organic or inorganic metal salt, such as halide (e.g. chloride, bromide or iodide), nitrate, sulfate or phosphate of, e.g., a transition metal such as copper, manganese, zinc or iron.

Examples of compounds of general formula (I), according to the present invention, are given in the following Table 1.

Table 1

| Compound | Ar | X, K, Z | $B_1$ | $B_2$ | R | A |
|---|---|---|---|---|---|---|
| 1 | 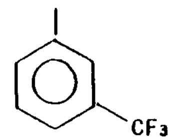 | 0, 0, 0 | $-CH_2CH_2-$ | $-CH_2CH_2-$ | $-CF_2CF_2H$ | 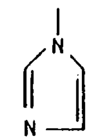 |
| 2 | 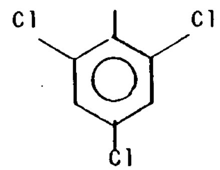 | 0, 0, 0 | $-CH_2CH_2-$ | $-CH_2CH_2-$ | $-CF_2CF_2H$ | 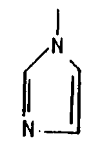 |
| 3 | 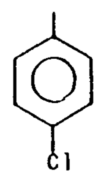 | 0, 0, 0 | $-CH_2CH_2-$ | $-CH_2CH_2-$ | $-CF_2CF_2H$ | 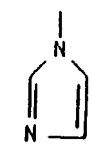 |
| 4 | 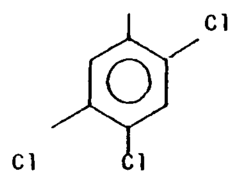 | 0, 0, 0 | $-CH_2CH_2-$ | $-CH_2CH_2-$ | $-CF_2CF_2H$ | 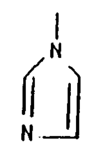 |

Table 1 (cont'd)

| Compound | Ar | X, K, Z | $B_1$ | $B_2$ | R | A |
|---|---|---|---|---|---|---|
| 5 | 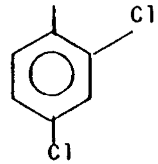 | 0, 0, 0 | $-CH_2CH_2-$ | $-CH_2CH_2-$ | $-CF_2CF_2H$ | 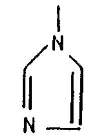 |
| 6 | 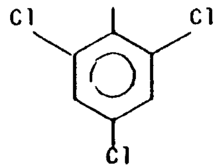 | 0, 0, 0 | $-CH_2CH_2-$ | $-CH_2CH_2-$<br>$CH_3$ | $-CF_2CF_2H$ | 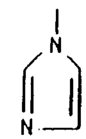 |
| 7 | 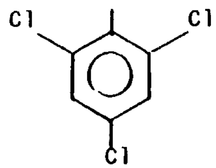 | 0, 0, 0 | $-CH_2CH_2-$ | $-CH_2CH_2CH_2-$ | $-CF_2CF_2H$ | 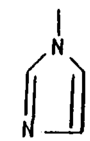 |
| 8 | 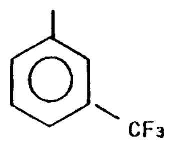 | 0, 0, 0 | $-CH_2CH_2-$ | $-CH_2CH_2-$ | $-CF_2CF.HOCF_3$ | 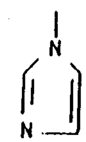 |

Table 1 (cont'd)

| Compound | Ar | X, K, Z | $B_1$ | $B_2$ | R | A |
|---|---|---|---|---|---|---|
| 9 | 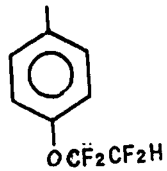 | 0, 0, 0 | $-CH_2CH_2-$ | $-CH_2CH_2-$ | $-CF_2CF_2H$ | 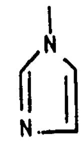 |
| 10 | 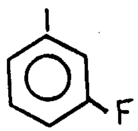 | 0, 0, 0 | $-CH_2CH_2-$ | $-CH_2CH_2-$ | $-CF_2CF_2H$ | 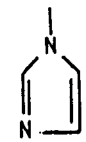 |
| 11 | 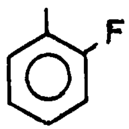 | 0, 0, 0 | $-CH_2CH_2-$ | $-CH_2CH_2-$ | $-CF_2CF_2H$ | 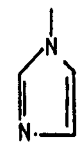 |
| 12 | 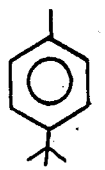 | 0, 0, 0 | $-CH_2CH_2-$ | $-CH_2CH_2-$ | $-CF_2CF_2H$ | 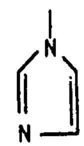 |
| 13 | 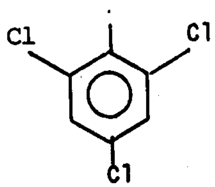 | 0, 0, 0 | $-CH_2CH_2-$ | $-CH_2CH_2-$ | $-CF_2CFClH$ | 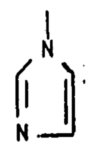 |

Table 1 (cont'd)

| Compound | Ar | X, K, Z | $B_1$ | $B_2$ | R | A |
|---|---|---|---|---|---|---|
| 14 | 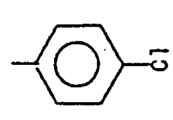 | O, O, O | $-CH_2CH_2-$ | $-CH_2CH_2-$ | $-CF_2CF_2H$ | 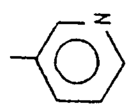 |

The compounds of general formula (I) may be prepared by several synthesis routes, some of which are summarized in the following schemes:

SYNTHESIS ROUTE A

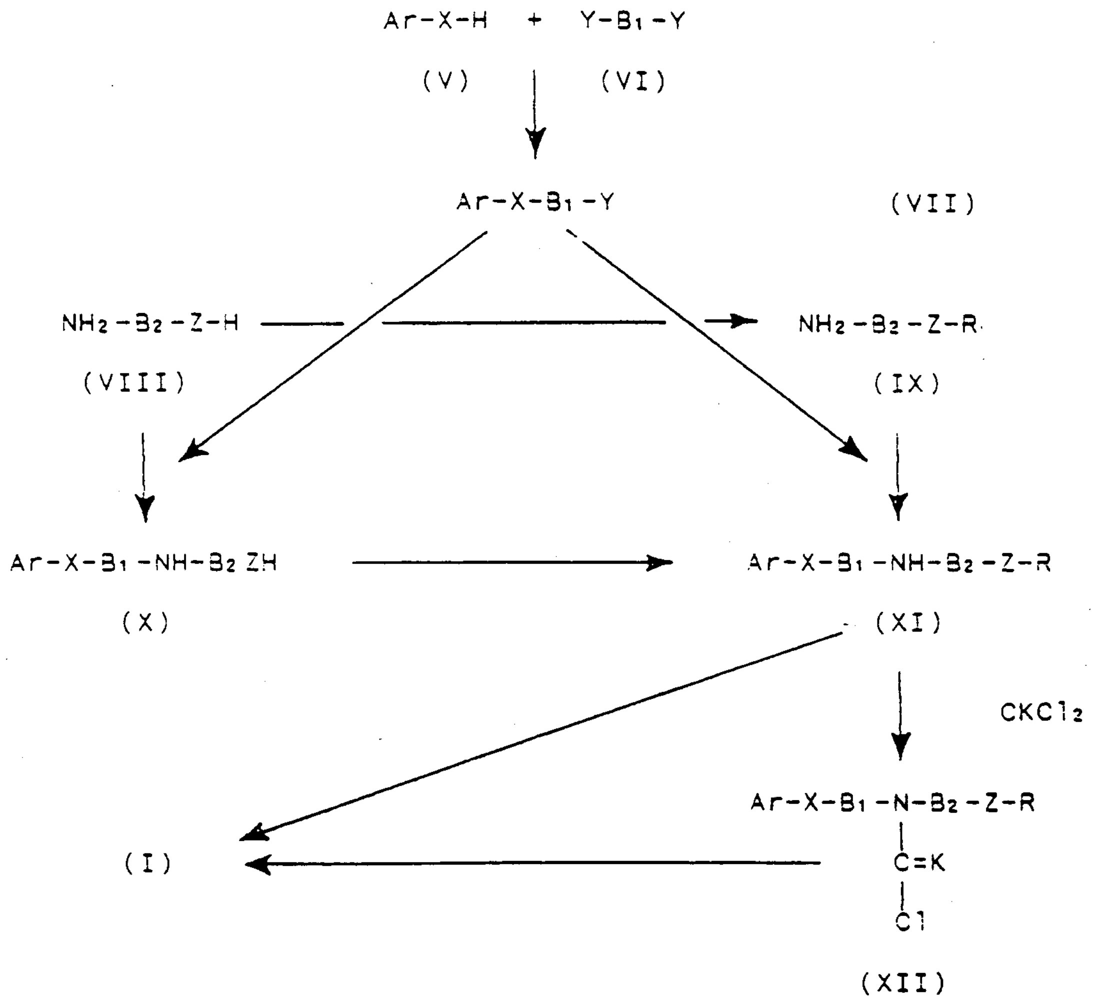


wherein:

Y = a leaving group, such as, e.g., halogen or a sulfonic acid ester.

According to above scheme (A), the compound (VII) is obtained through the reaction of compound (V) with the compound (VI), in the presence of a base, such as, e.g., sodium hydroxide or potassium hydroxide, sodium hydride, potassium tert.-butoxide, or of an alkali-metal carbonate to which a phase-transfer catalyst is added, in suitable solvents, such as, e.g., water or alcohols like methanol, ethanol, butanol, ethylene glycol, or, in case the reaction is carried out under phase-transfer conditions, in an aprotic polar solvent, such as, e.g., dimethylformamide, methyl-pyrrolidone, or in an aromatic solvent, such as toluene, or in a halogenated solvent, such as, e.g., methylene chloride or dichloroethane.

The reaction temperatures generally range from 0 °C to the boiling temperature of the solvent, as described in Organic Syntheses, coll. vol. I, page 435.

The compound (V) can also be used in salified form, e.g. with alkali or alkaline earth metals (Na, K, Mg, Ca, etc.).

The compound (VII) is the reacted with an amine (VIII) or with an amine (IX), in the presence of an inorganic or organic base, such as, e.g., an alkali metal (e.g. Na or K) hydroxide, carbonate or bicarbonate, triethylamine or pyridine, or by using an excess of the amine (VIII) or (IX) as acid acceptor, in solvents and under conditions which are analogous to those indicated for the previous step.

Depending on the amine used, the intermediate (X) or the intermediate (XI) will be obtained.

The intermediate compound (XI) is then reacted with a carbonyl derivative of the nitrogen containing heterocyclic compound from which the group A is derived (e.g. with carbonyl-diimidazole) in aromatic or halogenated solvents, at temperatures within the range of from 0 °C up to the boiling temperature of the solvent, in order to obtain the compound (I).

According to a different route, the compound (XI) is reacted with phosgene or thiophosgene in organic solvents, such as e.g. ethyl acetate. Thereby the compound (XII) is formed, from which, by subsequent reaction with a nitrogen containing heterocycle of the type A (for example imidazole), the compound (I) can be obtained.

According to an alternative route, the compound (XI) is reacted with the chloride of a nicotinic acid in order to obtain the compound (I).

The intermediate compound (XI) can also be obtained:

- by the addition reaction of the compound (X) to a polyfluoroolefin, for example a compound of formula:

  $CF_2 = CX_1X_2$ (XVIII)

  wherein:
  $X_1$ = Cl, F, $CF_3$, $OCF_3$ and
  $X_2$ = F, $CF_3$;
  in the presence of catalytic or stoichiometric amounts of strong bases, such as sodium hydride or potassium tert.-butoxide, in dipolar aprotic solvents, such as, e.g., dimethylsulfoxide, dimethylformamide, or in an alcoholic solvent, such as, e.g., tert.-butanol, at temperatures of from -20°C to 100°C;
- by means of the reaction of an alkali-metal (e.g. Na or K) salt of the compound (X) with compounds of the type Y-R,

wherein Y is a suitable leaving group, such as a halogen (e.g. Cl, Br or J) or a sulfonic ester.

Similarly, the amine (IX) can be obtained from the compound (VIII) by means of the same methods as described above for compound (XI).

The compounds which contain a group R in which at least one hydrogen atom and more than one halogen atom are present can be converted into the corresponding unsaturated compounds by means of a dehydrohalogenation reaction.

SYNTHESIS ROUTE (B)

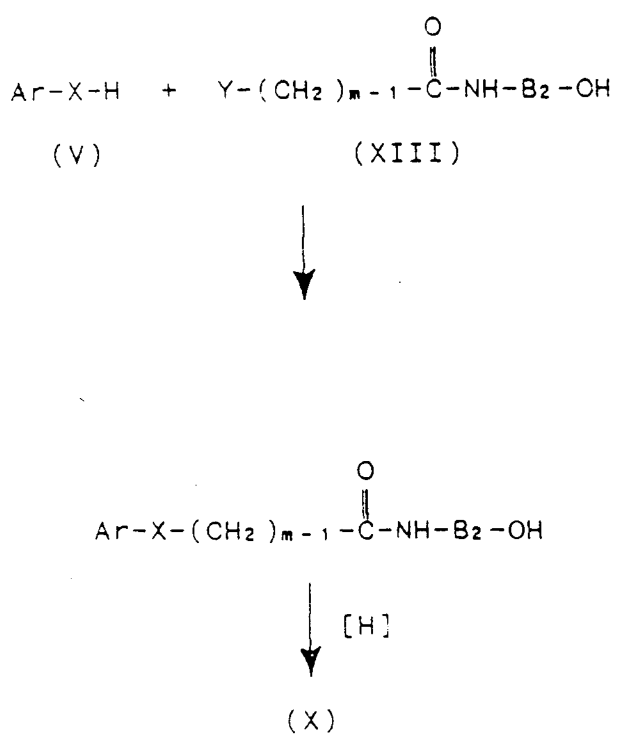

(V) (XIII)

(XIV)

(X)

wherein: m = 1 - 6.

According to the above synthesis route B, the amide (XIV) is obtained by reacting the compound (XIII) with the compound (V), in the presence of either organic or inorganic bases, in solvents like methanol,

ethanol, ethylene glycol, polyethylene glycols, dimethylformamide, or under phase-transfer conditions.

The compound (XIV) is subsequently reduced to yield the compound (X), e.g., by using lithium-aluminum hydride in solvents of the ether type, such as tetrahydrofuran, and the intermediate (X) is then converted into the compound (I) by following one of the routes shown in the above synthesis route A.

SYNTHESIS ROUTE C

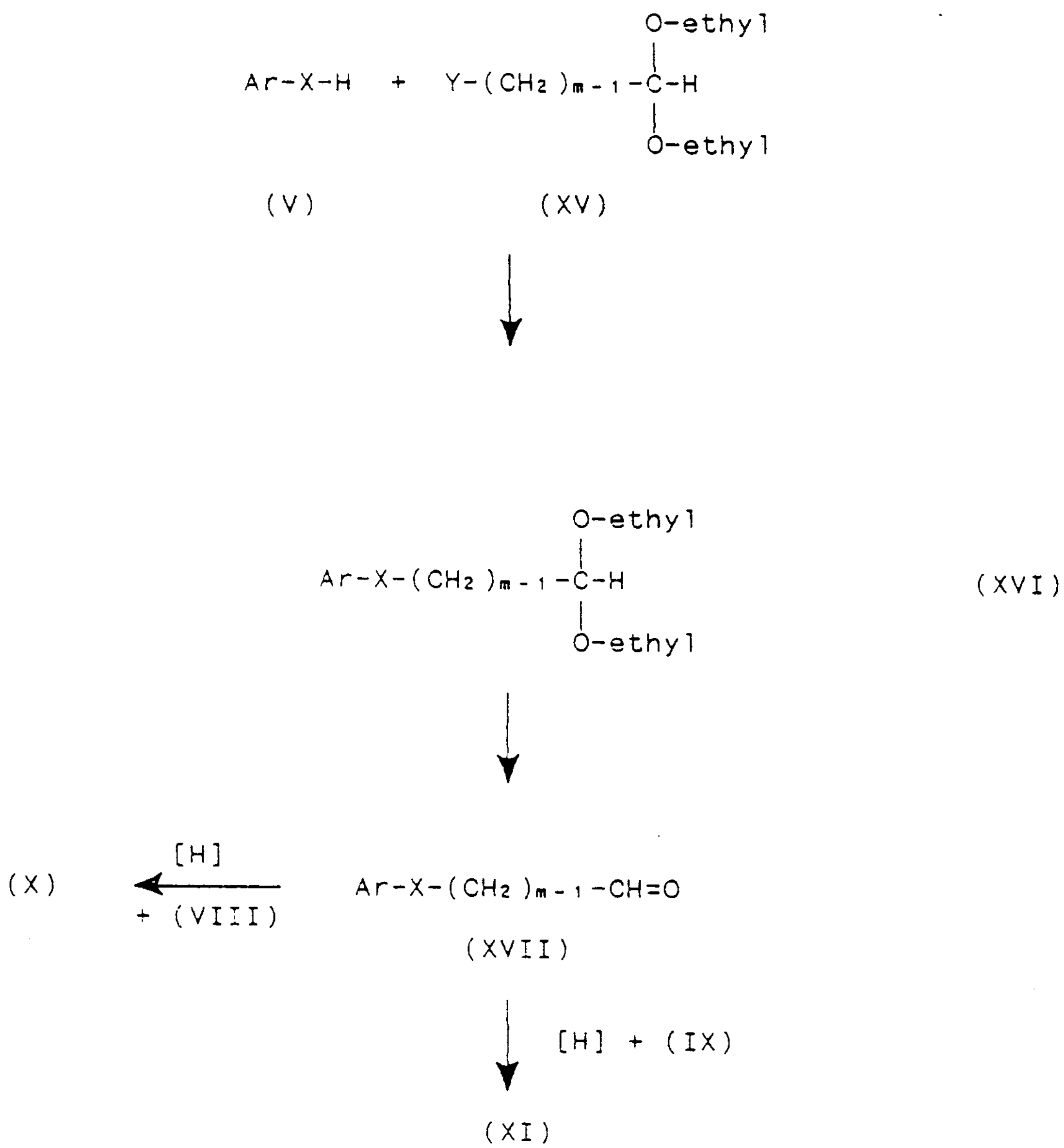


(V) (XV) (XVI) (X) (XVII) (XI)

wherein: m = 1 to 6.

According to synthesis route C, the acetal (XVI) is obtained by reacting a compound of formula (XV) with a compound of formula (V). From compound (XVI) the aldehyde (XVII) is obtained by means of a subsequent deprotection reaction; said aldehyde (XVII) is then reacted with the amine (VIII), in the presence of reducing systems, such as, e.g., hydrogen, and catalysts, e.g., Pt or Pd, in organic solvents such as, e.g., methyl alcohol, under neutral or acidic conditions, e.g. in the presence of sulfuric acid, to yield the compound (X) from which, by following routes like the ones shown in the above synthesis route A, the compound (I) may be obtained.

Alternatively, the aldehyde (XVII) is used for the reducing alkylation of the amine (IX), e.g., with sodium cyanoborohydride under the conditions known from the pertinent technical literature, in order to yield the compound (XI), which is then converted into the compound (I) by following one of the routes outlined above.

The compounds of general formula (I) are very powerful inhibitors of the growth of several species of pathogenic fungi which infect the cultivations of useful plants.

They show both preventive and curative activity when they are applied to useful plants or to parts thereof, such as, e.g., the leaves, and are particularly efficient in preventing the diseases caused by obliged pathogenic fungi, such as, e.g., those belonging to the genera Erysiphe and Puccinia.

Examples of plant diseases which can be controlled with the compounds according to the present invention are the following:

- Erysiphe graminis on cereals;
- Sphaeroteca fuliginea on cucurbits (e.g., cucumber);
- Puccinia on cereals;
- Septoria on cereals;
- Helminthosporium on cereals;
- Rhynchosporium on cereals;
- Podosphaera leucotricha on apple trees;
- Uncinula necator on vine;
- Venturia inaequalis on apple trees;
- Piricularia oryzae on rice;
- Botrytis cinerea;
- Fusarium on cereals;

and still further diseases.

For practical use in agriculture, it is often advantageous to have available fungicidal compositions containing one or more compounds of formula (I) as their active ingredient.

The application of these compositions may be carried out on any parts of the plants; for example, on leaves, stems, branches and roots, or on the seeds, before the sowing, or also on the soil in which the plant grows.

The compositions may, for instance, be used in the form of dry powders, wetting powders, emulsifiable concentrates, pastes, granulates, solutions, suspensions, etc. The selection of the type of composition will depend on the specific intended use. Said compositions are prepared in known manner, e.g., by diluting or dissolving the active substance with a solvent medium and/or a solid diluent, optionally in the presence of surfactants. As examples of solid diluents, or supports, the following may be mentioned: silica, kaolin, bentonite, talc, fossile flour, dolomite, calcium carbonate, magnesia, gypsum, clays, synthetic silicates, attapulgite, sepiolite. As liquid diluents, of course besides water, various types of solvents can be used, for example aromatic solvents (benzene, xylenes or blends of alkylbenzenes), chloroaromatic solvents (chlorobenzene), paraffins (petroleum fractions), alcohols (methanol, propanol, butanol), amines, amides (dimethylformamide), ketones (cyclohexanone, acetophenone, isophorone, ethylamyl-ketone) and esters (isobutyl acetate).

As surfactants sodium, calcium or triethanolamine salts of alkylsulfates, alkylsulfonates, alkyl-arylsulfonates, polyethoxylated alkylphenols, fatty alcohols condensed with ethylene oxide, polyethoxylated fatty acids, polyethoxylated sorbitol esters, polyethoxylated fats and lignin-sulfonates can, for instance, be used. The compositions can also contain special additives for particular purposes, e.g., bonding agents, such as gum arabic, polyvinyl alcohol and polyvinyl pyrrolidone.

If so desired, other compatible acitve substances, such as fungicides, plant protection products, phytoregulators, herbicides, insecticides and fertilizers can also be added to the compositions of the present invention.

The concentration of active substance in the above compositions can vary within a wide range, depending on the active compound, the culture, the pathogenic agent, the environmental conditions and the type of formulation used. In general, the concentration of active substance ranges from 0.1 to 95, preferably from 0.5 to 90% by weight.

The following examples are to illustrate the present invention.

Example 1

Synthesis of N-[2-(1,1,2,2-tetrafluoro-ethoxy)-ethyl]-N-[2-(3-trifluoromethyl-phenoxy)ethyl]-1-carboxyamido-imidazole (Compound 1)

To a solution of 1.3 grams of N-[2-(1,1,2,2-tetrafluoro-ethoxy)-ethyl]-N-[2-(3-trifluoromethyl-phenoxy)-ethyl]-amine in 9 ml of toluene, 0.73 g of carbonyl-diimidazole are added.

The mixture is kept under stirring for 8 hours at 80 °C and under a nitrogen blanket. After the removal of the reaction solvent by evaporation under reduced pressure, the residue is taken up in methylene chloride. The resulting solution is washed with water, dried over sodium sulfate and evaporated under reduced pressure. The resulting crude material is purified by means of chromatography on silica, a 95:5 (volume/volume = V/V) mixture of $CH_2Cl_2$:MeOH being used as the eluent.

Obtained are 1.2 g of an oil, the spectroscopic characteristics of which are consistent with those expected for compound 1.

$^1$H-NMR (60 MHz) in $CDCl_3$:
δ = 3.93 (2H, t)
3.96 (2H, t)
4.25 (4H, t)
6.13 (1H, tt)
7.00-7.50 (6H, m)
8.00 (1H, s).

Example 2

Synthesis of N-[2-(1,1,2,2-tetrafluoro-ethoxy)-ethyl]-N-[2-(3-trifluoromethyl-phenoxy)ethyl]-amine

To a solution of 2.0 grams of 2-[2-(3-trifluoro-methylphenoxy)-ethylamino]-ethanol in 14 ml of dimethylsulfoxide, cooled at 5 °C, 0.35 g of potassium tert.-butoxide is added. The reaction flask is then charged with tetrafluoroethylene whereupon a slight exotherm is observed. The reaction mixture is kept standing for a few hours under an atmosphere of said gas, whereupon the solution is quenched by pouring it into deionized water. Thereafter the whole mixture is extracted with methylene chloride. The organic phase is dried over sodium sulfate and is evaporated. 1.3 g of an oily residue are obtained.

$^1$H-NMR (60 MHz) in $CDCl_3$:
δ = 2.83 (2H, t)
2.90 (2H, t)
4.03 (4H, t)
5.70 (1H, tt)
7.00-7.50 (4H, m).

Example 3

Synthesis of 2-[2-(3-trifluoromethyl-phenoxy)ethylamino]-ethanol

17.7 g of 1-bromo-2-(3-trifluoro-methyl-phenoxy)-ethane are added dropwise to a solution of 14.1 g of ethanolamine in 33 ml of ethanol. The mixture is kept under stirring for 40 hours at room temperature. The solvent is then evaporated under reduced pressure and the residue is taken up in 5 N NaOH (26 ml). The obtained solution is extracted with methylene chloride, which subsequently is washed with water, dried over sodium sulfate and evaporated to dryness. A white crystalline solid is obtained which is taken up in hexane, filtered and washed with the same solvent. 12.2 g of product are obtained.

$^1$H-NMR (60 MHz) in $CDCl_3$:
δ = 2.95 (2H, t)
3.20 (2H, t)
3.90 (2H, t)
4.35 (2H, t)
7.00-7.50 (4H, m).

Example 4

Synthesis of 1-bromo-2-(3-trifluoromethylphenoxy)ethane

A solution of NaOH (33%, 17 ml) is added dropwise to a solution of 3-trifluoromethylphenol (2.5 g) and 1,2-dibromoethane (30.0 g) in deionized water (30 ml). The resulting mixture is kept under reflux conditions for 7 hours. The reaction mixture is then cooled, whereupon the formed oil is separated from the aqueous phase and is distilled under a pressure of 15 mm Hg. The fraction boiling in the range of from 118 to 120 °C is collected. 26 g of desired product are obtained.

$^1H$-NMR (60 MHz) in $CDCl_3$:
δ = 2.95 (2H, t)
3.20 (2H, t)
3.90 (2H, t)
4.35 (2H, t)
7.00-7.50 (4H, m).

Example 5

Synthesis of N-[2-(1,1,2,2-tetrafluoroethoxy)-ethyl]-N-[2-(2,4,6-trichlorophenoxy)-ethyl]-1-carboxyamido-imidazole (Compound 2)

The reaction is carried out in the same way as described in Example 1, starting from N-[2-(1,1,2,2-tetrafluoroethoxy)-ethyl]-N-[2-(2,4,6-trichlorophenoxy)-ethyl]-amine
$^1H$-NMR (60 MHz) in $CDCl_3$:
δ = 3.90 (4H, m)
4.20 (4H, m)
5.66 (1H, tt)
7.00-7.44 (3H, s)
7.90 (1H, s).

Example 6

Synthesis of N-[2-(1,1,2,2-tetrafluoroethoxy)-ethyl]-N-[2-(2,4,6-trichlorophenoxy)-ethyl]-amine

The reaction is carried out in the same way as described in Example 2, starting from 2-[2-(2,4,6-trichlorophenoxy)-ethyl)-amino]-ethanol
$^1H$-NMR (60 MHz) in $CDCl_3$:
δ = 2.95 (2H, t)
3.05 (2H, t)
4.10 (4H, t)
5,66 (1H, tt)
7.33 (2H, s).

Example 7

Synthesis of 2-[2-(2,4,6-trichlorophenoxy)-ethyl-amino]-ethanol.

The reaction is carried out in the same way as described in Example 3, starting from 1-bromo-2-(2,4,6-trichlorophenoxy)-ethane
$^1H$-NMR (60 MHz) in $CDCl_3$:
δ = 2.70 (2H, t)
2.87 (2H, t)
3.65 (2H, t)
4.40 (2H, t)
7.72 (2H, s).

Example 8

Synthesis of 1-bromo-2-(2,4,6-trichlorophenoxy)-ethane

The reaction is carried out in the same way as described in Example 4, starting from 2,4,6-trichlorophenol
$^1H$-NMR (60 MHz) in $CDCl_3$:
δ = 3.65 (2H, t)
4.30 (2H, t)
7.31 (2H, s).

Examples 9-19

By following the procedure described in Example 1, the following compounds were prepared by stating from the corresponding amines.

Compound 3:

N-[2-(1,1,2,2-tetrafluoroethoxy)-ethyl]-N-[2-(4-chlorophenoxy)-ethyl]-1-carboxyamido-imidazole

$^1$H-NMR (60 MHz) in $CDCl_3$ :
δ = 3.90 (4H, m)
4.20 (4H, m)
5.60 (1H, tt)
6.60-7.40 (4H, m)
8.00 (1H, s).

Compound 4:

N-[2-(1,1,2,2-tetrafluoroethoxy)-ethyl]-N-[2-(2,4,5-trichlorophenoxy)-ethyl]-1-carboxyamido-imidazole

$^1$H-NMR (60 MHz) in $CDCl_3$ :
δ = 3.95 (4H, m)
4.20 (4H, m)
5.70 (1H, tt)
6.96-7.50 (4H, m)
8.00 (1H, s).

Compound 5:

N-[2-(1,1,2,2-tetrafluoroethoxy)-ethyl]-N-[2-(2,4-dichlorophenoxy)-ethyl]-1-carboxyamido-imidazole

$^1$H-NMR (60 MHz) in $CDCl_3$ :
δ = 4.05 (4H, m)
4.30 (4H, m)
5.70 (1H, tt)
6.90-7.50 (5H, m)
8.00 (1H, s).

Compound 6:

N-[2-(1,1,2,2-tetrafluoroethoxy)-propyl]-N-[2-(2,4,6-trichloro-phenoxy)-ethyl]-1-carboxyamido-imidazole

$^1$H-NMR (60 MHz) in $CDCl_3$ :
δ = 1.35 (3H, d)
3.6-4.30 (7H, m)
5.70 (1H, tt)
7.50 (4H, m)
8.00 (1H, s).

Compound 7:

N-[3-(1,1,2,2-tetrafluoroethoxy)-propyl]-N-[2-(2,4,6-trichloro-phenoxy)-ethyl]-1-carboxyamido-imidazole

$^1$H-NMR (60 MHz) in $CDCl_3$ :
δ = 2.15 (2H, m)
3.80 (4H, m)
4.10 (4H, m)
5.65 (1H, tt)

7.00-7.40 (4H, m)
7.95 (1H, s).

Compound 8:

N-[2-(1,1,2-trifluoro-2-trifluoromethoxy-ethoxy)-ethyl]-N-[2-(3-trifluoromethylphenoxy)-ethyl]-1-carboxyamido-imidazole

$^1$H-NMR (60 MHz) in $CDCl_3$:
δ = 3.90 (4H, m)
4.60 (4H, m)
5.65 (1H, dt)
6.90-7.60 (6H, m)
8.00 (1H, s).

Compound 9:

N-[2-(1,1,2,2-tetrafluoroethoxy)-ethyl]-N-[2-(1,1,2,2-tetrafluoroethoxyphenoxy)ethyl]-1-carboxyamido-imidazole

$^1$H-NMR (60 MHz) in $CDCl_3$:
δ = 3.90 (4H, m)
4.20 (4H, m)
5.65 (1H, tt)
5.87 (1H, tt)
6.70-7.30 (5H, m)
7.97 (1H, s).

Compound 10:

N-[2-(1,1,2,2-tetrafluoroethoxy)ethyl]-N-[2-(3-fluorophenoxy) ethyl]-1-carboxyamido-imidazole

$^1$H-NMR (60 MHz) in $CDCl_3$:
δ = 3.80 (4H, m)
4.25 (4H, m)
5.75 (1H, tt)
6.50-7.55 (5H, m)
8.05 (1H, s)

Compound 11:

N-[2-(1,1,2,2-tetrafluoroethoxy)ethyl]-N-[2-(2-fluorophenoxy) ethyl]-1-carboxyamido-imidazole

$^1$N-NMR (60 MHz) in $CDCl_3$:
δ = 3.85 (4H, m)
4.15 (4H, m)
5.60 (1H, tt)
6.80-7.40 (5H, m)
7.93 (1H, s).

Compound 12:

N-[2-(1,1,2,2-tetrafluoroethoxy)ethyl]-N-[2-(4-tert.-butylphenoxy)ethyl]-1-carboxyamido-imidazole

$^1$H-NMR (60 MHz) in $CDCl_3$:
δ = 1.40 (9H, s)
3.87 (4H, m)
4.20 (4H, m)

5.60 (1H, tt)
6.75-7.45 (5H, m)
8.00 (1H, s).

Compound 13:

N-[2-(1,1,2-trifluoro-2-chloroethoxy)ethyl]-N-[2-(2,4,6-trichlorophenoxy)ethyl]-1-carboxyamido-imidazole

$^1$H-NMR (60 MHz) in $CDCl_3$:
$\delta$ = 4.00 (8H, m)
6.00 (1H, dt)
6.60-7.30 (6H, m)
7.90 (1H, s).

Example 20

Synthesis of N-[2-(1,1,2,2-tetrafluoroethoxy)-ethyl]-N-[2-(4-chlorophenoxy)ethyl]-3-carboxyamido-pyridine

Compound 14:

To a solution of N-[2-(1,1,2,2-tetrafluoroethoxy)-ethyl]-N-[2-(4-chlorophenoxy)-ethyl]amine (1.0 g) and nicotinoyl chloride (0.56 g) in methylene chloride (6.5 ml), triethylamine (6.9 g) is slowly added dropwise.

The reaction mixture is kept overnight with stirring at room temperature and is then treated with water. Thereafter the organic phase is separated, dried and evaporated under reduced pressure.

The mixture thus obtained is purified by chromatography on silica, using as eluent a 95:5 (V:V) mixture of $CH_2Cl_2$:MeOH. Obtained are 0.5 g of an oil, the spectroscopic characteristics of which are consistent with those expected for compound 15.
$^1$H-NMR (60 MHz) in $CDCl_3$:
$\delta$ = 3.90 (4H, m)
4.20 (4H, m)
5.75 (1H, tt)
6.60-8.00 (7H, m)
8.65 (1H, s).

Example 21

Determination of fungicidal activity on oidium of wheat (Erysiphe Graminis D.C.)

Preventive Activity:

Leaves of wheat cv. Irnerio, grown in pots inside a conditioned room, were treated by spraying both leaf faces with the products to be tested in water-acetone solution (20% (V/V) of acetone).

After 1 day in a conditioned room at 20 °C and 70% R.H., an aqueous suspension of Erysiphe graminis (200,000 conidia/ml) was sprayed on to both faces of the plant leaves.

After 24 hours in a humidity-saturated atmosphere of 21 °C the plants were left standing inside a conditioned room for the incubation of the fungus.

At the end of the incubation period (12 days), the extent of the infection was assessed visually, according to a scale ranging from 100 (healthy plant) to 0 (completely infected plant).

Curative Activity:

Leaves of wheat cv. Irnerio, grown in pots inside a conditioned room, were treated by spraying an aqueous suspension of Erysiphe graminis (200,000 conidia/ml) on both faces of all of the leaves.

After 24 hours in a humidity-saturated atmosphere of 21 °C, the leaves of the plants were treated by spraying the tested products in water-acetone solution (20% (V/V) of acetone) on both faces of all of the leaves.

At the end of the incubation period (12 days), the extent of the infection was assessed visually, according to a scale ranging from 100 (healthy plant) to 0 (completely infected plant).

The results are reported in Table 2.

Example 22

Determination of fungicide acitivity on linear rust of wheat (Puccinia graminis Pers.)

Preventive Activity:

Leaves of wheat cv. Irnerio, grown in pots inside a conditioned room, were treated by spraying the tested products in water-acetone solution (20% (V/V) of acetone) on both faces of all of the leaves.

After 1 day in a conditioned room at 23 °C and 70% R.H., a mixture of spores of Puccinia graminis in talc (100 mg of spores per 5 g of talc) was sprayed onto both faces of all plant leaves.

After 48 hours in a humidity-saturated atmosphere of 21 °C, the plants were stored in a conditioned room for fungus incubation.

At the end of the incubation time (14 days), the extent of the infection was assessed visually, according to a scale ranging from 100 (healthy plant) to 0 (completely infected plant).

Curative Activity:

Leaves of wheat cv. Irnerio, grown in pots inside a conditioned room, were treated by spraying a mixture of spores of Puccinia graminis in talc (100 mg of spores per 5 g of talc) on both faces of all of the leaves. After 48 hours in a humidity saturated atmosphere of 21 °C, the leaves were treated by spraying a water-acetone solution (20% (V/V) of acetone) of the tested products on both leaf faces.

At the end of the incubation period (14 days), the extent of the infection was assessed visually, according to a scale ranging from 100 (healthy plant) to 0 (completely infected plant).

The results are reported in Table 2.

Table 2

| Compound N. | Dose, g/l | Erysiphe graminis Wheat | | Puccinia graminis Wheat | |
|---|---|---|---|---|---|
| | | Preventive activity | Curative activity | Preventive activity | Curative activity |
| 1 | 1.0 | 97 | 100 | 80 | 100 |
| | 0.5 | 90 | 100 | 70 | 100 |
| | 0.125 | 85 | 95 | 40 | 90 |
| 2 | 1.0 | 100 | 100 | 60 | 95 |
| | 0.5 | 97 | 100 | 40 | 80 |
| | 0.125 | 90 | 100 | 20 | 40 |
| Ref.* | 1.0 | 97 | 100 | 40 | 60 |
| | 0.5 | 85 | 95 | 10 | 40 |
| | 0.125 | 60 | 85 | 0 | 10 |

* Ref. means reference compound, i.e. N-propyl-N-[2-(2,4,6-trichlorophenoxy)-ethyl]-1-carboxyamido-imidazole, known as Prochloraz (Sportak), see U.S. Patent 3,991,071

## Claims

### Claims for the following Contracting States : AT, BE, CH, DE, FR, GB, GR, LI, NL, SE

**1.** Compounds having the general formula:

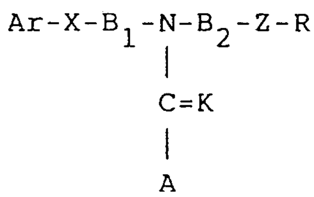

(I)

wherein:

| | |
|---|---|
| Ar | represents phenyl; phenyl substituted with one or more groups selected from halogen atoms, $C_{1-4}$-alkyl, $C_{1-4}$-haloalkyl, $C_{2-4}$-alkenyl, $C_{2-4}$-haloalkenyl, $C_{1-3}$-alkoxy and $C_{1-4}$-haloalkoxy groups; pyridyl; and pyridyl substituted with one or more groups selected from halogen atoms and $C_{1-3}$-haloalkyl groups; |
| K, X and Z | are, independently from each other, either O or S; |
| $B_1$ and $B_2$, | the same or different from each other, are linear or branched $C_{1-6}$-alkylene groups; |
| R | represents $C_{1-6}$-haloalkyl containing from 1 to 9 halogen atoms, $C_{2-8}$-haloalkenyl containing from 1 to 9 halogen atoms, $C_{3-8}$-haloalkoxyalkyl, $C_{3-8}$-haloalkoxyalkenyl, with the halogen being preferably fluorine; |
| A | represents a group selected from: |

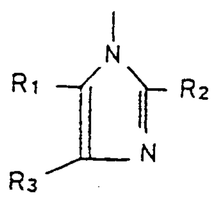
;
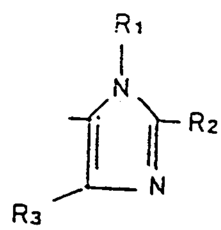
;
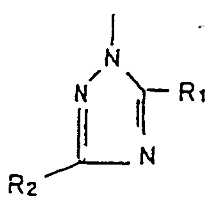
;
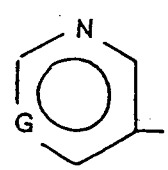

wherein:
$R_1$, $R_2$ and $R_3$ represent H; and
G is either CH or N;
and salts and metal complexes thereof.

**2.** Compounds according to claim 1, wherein

| | |
|---|---|
| Ar | is phenyl optionally substituted by 1 to 3 substituents selected from F, Cl and $CF_3$; and |
| K, | X and Z represent oxygen. |

**3.** Compounds according to any one of claims 1 and 2, wherein $B_1$ and $B_2$ independently represent ethylene or linear or branched propylene or butylene.

**4.** Compounds according to any one of claims 1 to 3, wherein A is selected from 1-imidazolyl, 5-imidazolyl and 3-pyridyl.

**5.** Compounds according to any one of claims 1 to 4, wherein R is a $C_{1-6}$ haloalkyl radical containing from 1 to 9 halogen atoms, preferably selected from F and Cl.

**6.** Compounds according to any one of claims 1 to 4, wherein R is selected from $-CF_2CF_2H$, $-CF_2CFHOCF_3$ and $-CF_2CFClH$.

**7.** Compounds according to claim 1, i.e. N-[2-(1,1,2,2-tetrafluoroethoxy)-ethyl]-N-[2-(3-trifluoromethyl-phenoxy) ethyl]-1-carboxyamido-imidazole,
N-[2-(1,1,2,2-tetrafluoroethoxy)-ethyl]-N-[2-(2,4,6-trichlorophenoxy)-ethyl]-1-carboxyamido-imidazole,
N-[2-(1,1,2,2-tetrafluoroethoxy)-ethyl]-N-[2-(4-chlorophenoxy)-ethyl]-1-carboxyamido-imidazole,
N-[2-(1,1,2,2-tetrafluoroethoxy)-ethyl]-N-[2-(2,4,5-trichlorophenoxy)-ethyl]-1-carboxyamido-imidazole,
N-[2-(1,1,2,2-tetrafluoroethoxy)-ethyl]-N-[2-(2,4-dichlorophenoxy)-ethyl]-1-carboxyamido-imidazole,
N-[2-(1,1,2,2-tetrafluoroethoxy)-propyl]-N-[2-(2,4,6-trichloro-phenoxy)-ethyl]-1-carboxyamido-imidazole,
N-[3-(1,1,2,2-tetrafluoroethoxy)-propyl]-N-[2-(2,4,6-trichloro-phenoxy)-ethyl]-1-carboxyamido-imidazole,
N-[2-(1,1,2-trifluoro-2-trifluoromethoxy-ethoxy)-ethyl]-N-[2-(3-trifluoromethylphenoxy)-ethyl]-1-carboxyamido-imidazole
and N-[2-(1,1,2,2-tetrafluoroethoxy)-ethyl]-N-[2-(4-chlorophenoxy)ethyl]-3-carboxyamido-pyridine.

**8.** Process for preparing compounds according to claim 1 wherein R = $CF_2$-$CHX_1X_2$, said process comprising the following steps:

a) reacting compounds of general formulae (VII) and (VIII) according to the reaction:

$$\underset{(VII)}{Ar\text{-}X\text{-}B_1\text{-}Y} + \underset{(VIII)}{NH_2\text{-}B_2\text{-}Z\text{-}H} \longrightarrow \underset{(X)}{Ar\text{-}X\text{-}B_1\text{-}NH\text{-}B_2\text{-}Z\text{-}H}$$

in an organic solvent and in the presence of a base, at temperatures of from 0 °C up to the reflux temperature of the solvent;

b) reacting the compound of formula (X) resulting from step (a) with a compound of formula(XVIII) according to the reaction:

$$(X) + \underset{(XVIII)}{CF_2{=}CX_1X_2} \longrightarrow \underset{(XIa)}{Ar\text{-}X\text{-}B_1\text{-}NH\text{-}B_2\text{-}Z\text{-}CF_2\text{-}CHX_1X_2}$$

in dipolar aprotic or alcoholic solvents and in the presence of a strong base, at temperatures of from -20 °C to 100 °C;

c) reacting the resulting compound of formula (XIa) with a carbonyl derivative of A in aromatic or halogenated solvents and at temperatures of from 0 °C up to the reflux temperature of the solvent;

| | |
|---|---|
| Ar, X, Z, $B_1$, $B_2$ and A | having the meanings specified in claim 1; |
| Y | being halogen; |
| $X_1$ | being Cl, F, $CF_3$ or $OCF_3$; and |
| $X_2$ | being F or $CF_3$. |

**9.** Method for controlling fungus infections in useful plants, comprising the application of an efficient amount of a compound according to any one of claims 1 to 7, as such or as a suitable composition, onto the plant, the seeds and/or the surrounding soil, when the fungus infection is expected or has already occurred.

**10.** Fungicidal composition, containing as at least one of its active ingredients one or more compounds according to any one of claims 1 to 7, together with a solid or liquid carrier, and, if desired, other additives.

**Claims for the following Contracting State : ES**

**1.** Process for preparing compounds having the general formula:

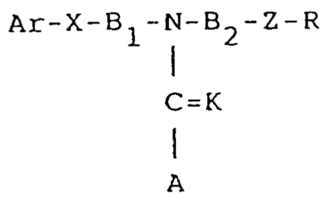

(I)

wherein:

| | |
|---|---|
| Ar | represents phenyl; phenyl substituted with one or more groups selected from halogen atoms, $C_{1-4}$-alkyl, $C_{1-4}$-haloalkyl, $C_{2-4}$-alkenyl $C_{2-4}$-haloalkenyl, $C_{1-3}$-alkoxy and $C_{1-4}$-haloalkoxy groups; pyridyl; and pyridyl substituted with one or more groups selected from halogen atoms and $C_{1-3}$-haloalkyl groups; |
| K, X and Z | are, independently from each other, either O or S; |
| $B_1$ and $B_2$, | the same or different from each other, are linear or branched $C_{1-6}$-alkylene groups; |
| R | is $CF_2$-$CHX_1X_2$; |
| $X_1$ | being Cl , F, $CF_3$ or $OCF_3$; and |
| $X_2$ | being F or $CF_3$; |
| A | represents a group selected from: |

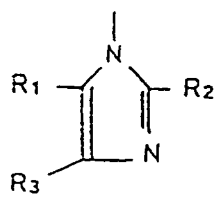 ; 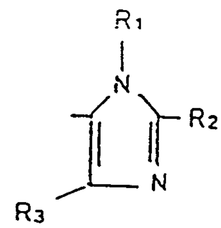 ;

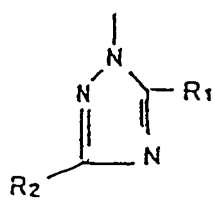 ; 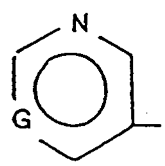

wherein:
$R_1$, $R_2$ and $R_3$ represents H; and
G is either CH or N;
and salts and metal complexes thereof; and
said process comprises the following steps:

a) reacting compounds of general formulae (VII) and (VIII) according to the reaction:

$$\underset{(VII)}{Ar\text{-}X\text{-}B_1\text{-}Y} + \underset{(VIII)}{NH_2\text{-}B_2\text{-}Z\text{-}H} \longrightarrow \underset{(X)}{Ar\text{-}X\text{-}B_1\text{-}NH\text{-}B_2\text{-}Z\text{-}H}$$

in an organic solvent and in the presence of a base, at temperatures of from 0 °C up to the reflux temperature of the solvent;

b) reacting the compound of formula (X) resulting from step (a) with a compound of formula (XVIII) according to the reaction:

$$(X) + CF_2{=}CX_1X_2 \longrightarrow Ar\text{-}X\text{-}B_1\text{-}NH\text{-}B_2\text{-}Z\text{-}CF_2\text{-}CHX_1X_2$$

(XVIII) (XIa)

in dipolar aprotic or alcoholic solvents and in the presence of a strong base, at temperatures of from -20 °C to 100 °C;

c) reacting the resulting compound of formula (XIa) with a carbonyl derivative of A in aromatic or halogenated solvents and at temperatures of from 0 °C up to the reflux temperature of the solvent;

Y being halogen.

**2.** Process according to claim 1, wherein

Ar is phenyl optionally substituted by 1 to 3 substituents selected from F, Cl and $CF_3$; and

K, X and Z represent oxygen.

**3.** Process according to any one of the claims 1 and 2, wherein $B_1$ and $B_2$ independently represent ethylene or linear or branched propylene or butylene.

**4.** Process according to any one of the claims 1 to 3, wherein A is selected from 1-imidazolyl, 5-imidazolyl and 3-pyridyl.

**5.** Process according to claim 1, wherein the compounds of formula (I) are N-[2-(1,1,2,2-tetrafluoroethoxy)-ethyl]-N-[2-(3-trifluoromethyl-phenoxy) ethyl]-1-carboxyamido-imidazole,
N-[2-(1,1,2,2-tetrafluoroethoxy)-ethyl]-N-[2-(2,4,6-trichlorophenoxy)-ethyl]-1-carboxyamido-imidazole,
N-[2-(1,1,2,2-tetrafluoroethoxy)-ethyl]-N-[2-(4-chlorophenoxy)-ethyl]-1-carboxyamido-imidazole,
N-[2-(1,1,2,2-tetrafluoroethoxy)-ethyl]-N-[2-(2,4,5-trichlorophenoxy)-ethyl]-1-carboxyamido-imidazole,
N-[2-(1,1,2,2-tetrafluoroethoxy)-ethyl]-N-[2-(2,4-dichlorophenoxy)-ethyl]-1-carboxyamido-imidazole,
N-[2-(1,1,2,2-tetrafluoroethoxy)-propyl]-N-[2-(2,4,6-trichloro-phenoxy)-ethyl]-1-carboxyamido-imidazole,
N-[3-(1,1,2,2-tetrafluoroethoxy)-propyl]-N-[2-(2,4,6-trichloro-phenoxy)-ethyl]-1-carboxyamido-imidazole,
N-[2-(1,1,2-trifluoro-2-trifluoromethoxy-ethoxy)-ethyl]-N-[2-(3-trifluoromethylphenoxy)-ethyl]-1-carboxyamido-imidazole
and N-[2-(1,1,2,2-tetrafluoroethoxy)-ethyl]-N-[2-(4-chlorophenoxy)ethyl]-3-carboxyamido-pyridine.

**6.** Fungicidal composition, containing as at least one of its active ingredients one or more compounds produced according to any one of claims 1 to 5, together with a solid or liquid carrier, and, if desired, other additives.

## Patentansprüche

## Patentansprüche für folgende Vertragsstaaten : AT, BE, CH, DE, FR, GB, GR, LI, NL, SE

**1.** Verbindungen der allgemeinen Formel:

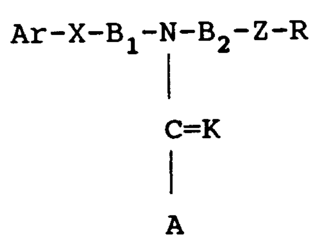

(I)

worin:

Ar bedeutet: Phenyl; Phenyl, substituiert mit einer oder mehreren Gruppen, ausgewählt aus Halogenatomen, $C_{1-4}$-Alkyl, $C_{1-4}$-Halogenalkyl, $C_{2-4}$-Alkenyl, $C_{2-4}$-Halogenalkenyl, $C_{1-3}$-Alkoxy und $C_{1-4}$-Halogenalkoxygruppen; Pyridyl; und Pyridyl, substituiert mit einer oder mehreren Gruppen, ausgewählt aus Halogenatomen und $C_{1-3}$-

Halogenalkylgruppen;

K, X und Z, unabhängig voneinander, entweder O oder S sind;

$B_1$ und $B_2$, die gleich oder verschieden sein können, lineare oder verzweigte $C_{1-6}$-Alkylengruppen sind;

R bedeutet: $C_{1-6}$-Halogenalkyl mit 1 bis 9 Halogenatomen, $C_{2-8}$-Halogenalkenyl mit 1 bis 9 Halogenatomen, $C_{3-8}$-Halogenalkoxyalkyl, $C_{3-8}$-Halogenalkoxyalkenyl, wobei das Halogen vorzugsweise Fluor ist;

A bedeutet eine Gruppe, ausgewählt aus:

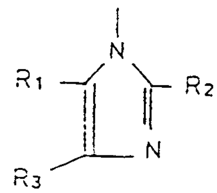
;
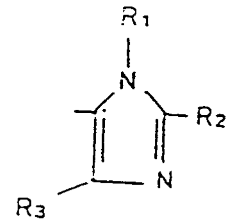
;
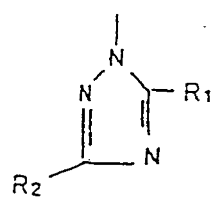
;
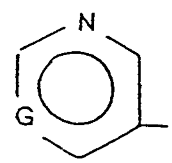

worin:
$R_1$, $R_2$ und $R_3$ H bedeuten; und
G entweder CH oder N ist;
und Salze und Metallkomplexe von diesen.

**2.** Verbindungen nach Anspruch 1, worin
Ar = Phenyl ist, gegebenenfalls mit 1 bis 3 Substituenten, ausgewählt aus F, Cl und $CF_3$ substituiert; und
K, X und Z Sauerstoff bedeuten.

**3.** Verbindungen nach einem der Ansprüche 1 und 2, worin $B_1$ und $B_2$ unabhängig voneinander Ethylen oder lineares oder verzweigtes Propylen oder Butylen bedeuten.

**4.** Verbindungen nach einem der Ansprüche 1 bis 3, worin A ausgewählt ist aus 1-Imidazolyl, 5-Imidazolyl und 3-Pyridyl.

**5.** Verbindungen nach einem der Ansprüche 1 bis 4, worin R ein $C_{1-6}$-Halogenalkylradikal mit 1 bis 9 Halogenatomen, vorzugsweise ausgewählt aus F und Cl, ist.

**6.** Verbindungen nach einem der Ansprüche 1 bis 4, worin R ausgewählt ist aus $-CF_2CF_2H$, $-CF_2CFHOCF_3$ und $-CF_2CFClH$.

**7.** Verbindungen nach Anspruch 1, und zwar N-[2-(1,1,2,2-Tetrafluorethoxy)-ethyl]-N-[2-(3-trifluormethylphenoxy)ethyl]-1-carboxyamido-imidazol,
N-[2-(1,1,2,2-Tetrafluorethoxy)-ethyl]-N-[2-(2,4,6-trichlorphenoxy)-ethyl]-1-carboxyamido-imidazol,
N-[2-(1,1,2,2-Tetrafluorethoxy)-ethyl]-N-[2-(4-chlorphenoxy)-ethyl]-1-carboxyamido-imdazol,
N-[2-(1,1,2,2-Tetrafluorethoxy)-ethyl]-N-[2-(2,4,5-trichlorphenoxy)-ethyl]-1-carboxyamido-imidazol,
N-[2-(1,1,2,2-Tetrafluorethoxy)ethyl]-N-[2-(2,4-dichlorphenoxy)-ethyl]-1-carboxyamido-imidazol,
N-(2-[1,1,2,2-Tetrafluorethoxy)-propyl]-N-[2-(2,4,6-trichlor-phenoxy)-ethyl]-1-carboxyamido-imidazol,
N-[3-(1,1,2,2-Tetrafluorethoxy)-propyl)-N-[2-(2,4,6-trichlorphenoxy)-ethyl]-1-carboxyamido-imidazol,
N-[2-(1,1,2-Trifluor-2-trifluormethoxy-ethoxy)-ethyl]-N-[2-(3-trifluormethylphenoxy)-ethyl)-1-carboxyamidoimidazol und

N-[2-(1,1,2,2-Tetrafluorethoxy]-ethyl)-N-[2-(4-chlorphenoxy)-ethyl]-3-carboxyamido-pyridin.

8. Verfahren zur Herstellung der Verbindungen nach Anspruch 1, worin R = $CF_2$-$CHX_1X_2$ ist, und das Verfahren die folgenden Schritte umfaßt:
a) Umsetzung der Verbindungen der allgemeinen Formeln (VII) und (VIII) nach folgender Reaktion:

$$\underset{(VII)}{Ar\text{-}X\text{-}B_1\text{-}Y} + \underset{(VIII)}{NH_2\text{-}B_2\text{-}Z\text{-}H} \longrightarrow \underset{(X)}{Ar\text{-}X\text{-}B_1\text{-}NH\text{-}B_2\text{-}Z\text{-}H}$$

in einem organischen Lösungsmittel und in Anwesenheit einer Base bei Temperaturen von 0° C bis zur Rückflußtemperatur des Lösungsmittels;
b) Umsetzung der Verbindung der Formel (X) aus Schritt
a) mit einer Verbindung der Formel (XVIII) nach folgender Reaktion:

$$(X) + \underset{(XVIII)}{CF_2{=}CX_1X_2} \longrightarrow \underset{(XIa)}{Ar\text{-}X\text{-}B_1\text{-}NH\text{-}B_2\text{-}Z\text{-}CF_2\text{-}CHX_1X_2}$$

in dipolaren aprotischen oder alkoholischen Lösungsmitteln und in Anwesenheit einer starken Base bei Temperaturen von -20° C bis 100° C;
c) Umsetzung der erhaltenen Verblndung der Formel (XIa) mit einem Carbonyl-Derivat von A in aromatischen oder halogenierten Lösungsmitteln bei Temperaturen von 0° C bis zur Rückflußtemperatur des Lösungsmittels; Ar, X, Z, $B_1$ $B_2$ und A haben die in Anspruch 1 definierten Bedeutungen;
Y ist Halogen;
$X_1$ ist Cl, F, $CF_3$ oder $OCF_3$; und
$X_2$ ist F oder $CF_3$.

9. Verfahren zur Bekämpfung von Pilzinfektionen bei Nutzpflanzen, welches die Anwendung einer wirksamen Menge einer Verbindung nach einem der Ansprüche 1 bis 7 als solche oder als geeignete Zusammensetzung auf die Pflanze, die Samen und/oder die umgebende Erde, wenn die Pilzinfektion erwartet wird oder bereits aufgetreten ist, umfaßt.

10. Fungizide Zusammensetzung, die als wenigstens einen ihrer aktiven Bestandteile eine oder mehrere Verbindungen nach einem der Ansprüche 1 bis 7, zusammen mit einem festen oder flüssigen Träger und gegebenenfalls anderen Additiven, umfaßt.

**Patentansprüche für folgenden Vertragsstaat : ES**

1. Verfahren zur Herstellung der Verbindungen der allgemeinen Formel:

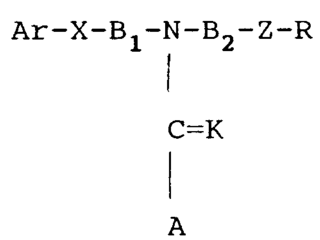

(I)

worin:

Ar bedeutet: Phenyl; Phenyl, substituiert mit einer oder mehreren Gruppen, ausgewählt aus Halogenatomen, $C_{1-4}$-Alkyl, $C_{1-4}$-Halogenalkyl, $C_{2-4}$-Alkenyl, $C_{2-4}$-Halogenal-

kenyl, $C_{1-3}$-Alkoxy und $C_{1-4}$-Halogenalkoxygruppen; Pyridyl; und Pyridyl, substituiert mit einer oder mehreren Gruppen, ausgewählt aus Halogenatomen und $C_{1-3}$-Halogenalkylgruppen;

K, X und Z, unabhängig voneinander, entweder O oder S sind;

$B_1$ und $B_2$, die gleich oder verschieden sein können, lineare oder verzweigte $C_{1-6}$-Alkylengruppen sind;

R = $CF_2$-$CHX_1X_2$ ist;

$X_1$ = Cl, F, $CF_3$ oder $OCF_3$ ist; und

$X_2$ = F oder $CF_3$ ist;

A eine Gruppe bedeutet ausgewählt aus:

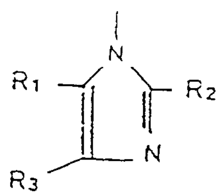 ; 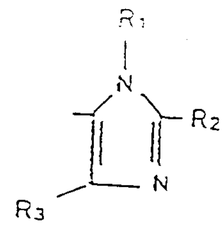 ;

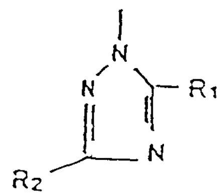 ; 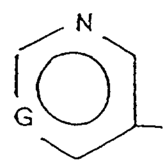

worin:
$R_1$, $R_2$ und $R_3$ H bedeuten; und
G entweder CH oder N ist;
und Salze und Metallkomplexe von diesen; und
wobei das genannte Verfahren die folgenden Schritte umfaßt:

a) Umsetzung der Verbindungen der allgemeinen Formeln (VII) und (VIII) nach folgender Reaktion:

$$\underset{(VII)}{Ar\text{-}X\text{-}B_1\text{-}Y} + \underset{(VIII)}{NH_2\text{-}B_2\text{-}Z\text{-}H} \longrightarrow \underset{(X)}{Ar\text{-}X\text{-}B_1\text{-}NH\text{-}B_2\text{-}Z\text{-}H}$$

in einem organischen Lösungsmittel und in Anwesenheit einer Base bei Temperaturen von 0° C bis zur Rückflußtemperatur des Lösungsmittels;

b) Umsetzung der Verbindung der Formel (X) aus Schritt

a) mit einer Verbindung der Formel (XVIII) nach folgender Reaktion:

$$(X) + \underset{(XVIII)}{CF_2{=}CX_1X_2} \longrightarrow \underset{(XIa)}{Ar\text{-}X\text{-}B_1\text{-}NH\text{-}B_2\text{-}Z\text{-}CF_2\text{-}CHX_1X_2}$$

in dipolaren aprotischen oder alkoholischen Lösungsmitteln und in Anwesenheit einer starken Base bei Temperaturen von -20° C bis 100° C;

c) Umsetzung der erhaltenen Verbindung der Formel (XIa) mit einem Carbonyl-Derivat von A in aromatischen oder halogenierten Lösungsmitteln bei Temperaturen von 0° C bis zur Rückflußtemperatur des Lösungsmittels; wobei Y Halogen ist.

**2.** Verfahren nach Anspruch 1, worin

Ar = Phenyl, gegebenenfalls substituiert mit 1 bis 3 Substituenten, ausgewählt aus F, Cl

und $CF_3$ ist; und
K, X und Z Sauerstoff bedeuten.

**3.** Verfahren nach einem der Ansprüche 1 und 2, worin $B_1$ und $B_2$ unabhängig voneinander Ethylen oder lineares oder verzweigtes Propylen oder Butylen bedeuten.

**4.** Verfahren nach einen der Ansprücje 1 bis 3. worin A ausgewählt ist aus 1-Imidazolyl, 5-Imidazolyl und 3-Pyridyl.

**5.** Verfahren nach Anspruch 1, worin die Verbindungen Formel (I) sind:
N-[2-(1,1,2,2-Tetrafluorethoxy)-ethyl]-N-[2-(3-trifluormethyl-phenoxy)ethyl]-1-carboxyamido-imidazol,
N-[2-(1,1,2,2-Tetrafluorethoxy)-ethyl]-N-[2-(2,4,6-trichlorphenoxy)-ethyl]-1-carboxyamido-imidazol,
N-[2-(1,1,2,2-Tetrafluorethoxy)-ethyl]-N-[2-(4-chlorphenoxy)-ethyl]-1-carboxyamido-imdazol,
N-[2-(1,1,2,2-Tetrafluorethoxy)-ethyl]-N-[2-(2,4,5-trichlorphenoxy)-ethyl]-1-carboxyamido-imidazol,
N-[2-(1,1,2,2-Tetrafluorethoxy)ethyl]-N-[2-(2,4-dichlorphenoxy)-ethyl]-1-carboxyamido-imidazol,
N-(2-[1,1,2,2-Tetrafluorethoxy)-propyl]-N-[2-(2,4,6-trichlor-phenoxy)-ethyl]-1-carboxyamido-imidazol,
N-[3-(1,1,2,2-Tetrafluorethoxy)-propyl]-N-[2-(2,4,6-trichlorphenoxy)-ethyl]-1-carboxyamido-imidazol,
N-[2-(1,1,2-Trifluor-2-trifluormethoxy-ethoxy)-ethyl]-N-[2-(3-trifluormethylphenoxy)-ethyl]-1-carboxyamidoimidazol und
N-[2-(1,1,2,2-Tetrafluorethoxy]-ethyl]-N-[2-(4-chlorphenoxy)-ethyl]-3-carboxyamido-pyridin.

**6.** Fungizide Zusammensetzung, die als wenigstens einen ihrer aktiven Bestandteile eine oder mehrere Verbindungen, hergestellt nach einem der Ansprüche 1 bis 5 zusammen mit einem festen oder flüssigen Träger und gegebenenfalls anderen Additiven, enthält.

**Revendications**
**Revendications pour les Etats contractants suivants : AT, BE, CH, DE, FR, GB, GR, LI, NL, SE**

**1.** Composés représentés par la formule générale:

```
Ar-X-B1-N-B2-Z-R
        '
        C=K              (I)
        '
        A
```

dans laquelle:

Ar représente un radical phényle; un radical phényle substitué par un ou plusieurs groupes sélectionnés parmi: atomes d'halogène, radical alkyle en $C_1$ à $C_4$, radical haloalkyle en $C_1$ à $C_4$, radical alkényle en $C_2$ à $C_4$, radical haloalkényle en $C_2$ à $C_4$, radical alcoxy en $C_1$ à $C_3$, ou radical haloalcoxy en $C_1$ à $C_4$; un radical pyridyle; et un radical pyridyle substitué par un ou plusieurs groupes sélectionnés parmi: atomes d'halogènes et radicaux haloalkyle en $C_1$ à $C_3$;
K, X et Z représentent indépendamment les uns des autres, un atome d'oxygène ou un atome de soufre;
$B_1$ et $B_2$, identiques ou différents l'un de l'autre, représentent des radicaux alkylène en $C_1$ à $C_6$, ramifiés ou linéaires;
R représente un radical haloalkyle en $C_1$ à $C_6$ contenant entre 1 et 9 atomes d'halogène, un radical haloalkényle en $C_2$ à $C_8$ contenant entre 1 et 9 atomes d'halogène, un radical haloalcoxyalkyle en $C_3$ à $C_8$, un radical haloalcoxyalkényle en $C_3$ à $C_8$, l'atome d'halogène étant de préférence un atome de fluor;
A représente un groupe sélectionné parmi:

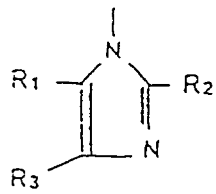 ;

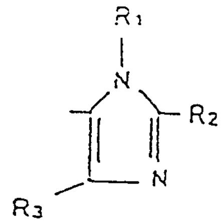 ;

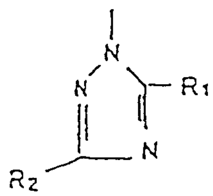 ;

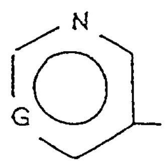

dans lesquelles:

$R_1$, $R_2$ et $R_3$ représentent H; et

G représente CH ou N;

ainsi que les sels et les complexes métalliques de ces composés.

**2.** Composés selon la revendication 1, caractérisés en ce que:

Ar représente un radical phényle, éventuellement substitué par 1 à 3 substituants sélectionnés parmi F, Cl et $CF_3$; et

K, X et Z représentent un atome d'oxygène.

**3.** Composés selon l'une quelconque des revendications 1 et 2, caractérisés en ce que $B_1$ et $B_2$ représentent indépendamment l'un de l'autre, un radical éthylène ou un radical butylène ou propylène, linéaire ou ramifié.

**4.** Composés selon l'une quelconque des revendications 1 à 3, caractérisés en ce que A est sélectionné parmi les radicaux 1-imidazolyle, 5-imidazolyle et 3-pyridyle.

**5.** Composés selon l'une quelconque des revendications 1 à 4, caractérisés en ce que R représente un radical haloalkyle en $C_1$ à $C_6$, contenant de 1 à 9 atomes d'halogène, qui sont de préférence sélectionnés parmi F et Cl.

**6.** Composés selon l'une quelconque des revendications 1 à 4, caractérisés en ce que R est sélectionné parmi $-CF_2CF_2H$, $-CF_2CFHOCF_3$ et $-CF_2CFClH$.

**7.** Composés selon la revendication 1, c'est-à-dire:

. N-[2-(1,1,2,2-tétrafluoroéthoxy)-éthyl]-N-[2-(3-trifluorométhylphénoxy)éthyl]-1-carboxyamido-imidazole,

. N-[2-(1,1,2,2-tétrafluoroéthoxy)-éthyl]-N-[2-(2,4,6-trichlorophénoxy)éthyl]-1-carboxyamido-imidazole,

. N-[2-(1,1,2,2-tétrafluoroéthoxy)-éthyl]-N-[2-(2,4,5-trichlorophénoxy)éthyl]-1-carboxyamido-imidazole,

. N-[2-(1,1,2,2-tétrafluoroéthoxy)-éthyl]-N-[2-(2,4-dichlorophénoxy)éthyl]-1-carboxyamido-imidazole,

. N-[2-(1,1,2,2-tétrafluoroéthoxy)-propyl]-N-[2-(2,4,6-trichlorophénoxy)éthyl]-1-carboxyamido-imidazole,

. N-[3-(1,1,2,2-tétrafluoroéthoxy)-propyl]-N-[2-(2,4,6-trichlorophénoxy)éthyl]-1-carboxyamido-imidazole,

. N-[2-(1,1,2-trifluoro-2-trifluorométhoxyéthoxy)-éthyl]-N-[2-(3-trifluorométhylphénoxy)éthyl]-1-carboxyamidoimidazole, et

. N-[2-(1,1,2,2-tétrafluoroéthoxy)-éthyl]-N-[2-(4-chlorophénoxy)-éthyl]-1-carboxyamido-pyridine.

**8.** Procédé de préparation des composés selon la revendication 1, caractérisé en ce que R = $CF_2$-$CHX_1X_2$, ledit procédé comprenant les étapes suivantes:

a) la réaction des dérivés de formule générale (VII) et (VIII) selon la réaction:

$$\underset{(VII)}{Ar-X-B_1-Y} + \underset{(VIII)}{NH_2-B_2-Z-H} \longrightarrow \underset{(X)}{Ar-X-B_1-NH-B_2-Z-H}$$

dans un solvant organique et en présence d'une base à des températures de 0 °C à la température de reflux du solvant;

b) la réaction du composé de formule (X) qui résulte de l'étape (a) avec un dérivé de formule (XVIII) selon la réaction:

$$(X) + \underset{(XVIII)}{CF_2=CX_1X_2} \longrightarrow \underset{(XIa)}{Ar-X-B_1-NH-B_2-Z-CF_2-CHX_1X_2}$$

dans des solvants alcooliques ou aprotiques dipolaires et en présence d'une base forte à des températures de -20 °C à 100 °C;

c) la réaction du composé de formule (XIa) résultant avec un dérivé carbonyle de A dans des solvants aromatiques ou halogénés à des températures de 0 °C à la température de reflux du solvant;

| | |
|---|---|
| Ar, X, Z, $B_1$, $B_2$ et A | étant tels que définis dans la revendication 1; |
| Y | représentant un atome d'halogène; |
| $X_1$ | représentant Cl, F, $CF_3$ ou $OCF_3$; et |
| $X_2$ | représentant F ou $CF_3$. |

**9.** Procédé de contrôle de l'infection de plantes utiles par des champignons comprenant l'application d'une quantité efficace d'un composé selon l'une quelconque des revendications 1 à 7, en tant que tel ou sous la forme d'une composition appropriée, sur les plantes, les graines et/ou le sol environnant au moment où l'infection va se produire ou après que l'infection se soit déjà produite.

**10.** Composition fongicide dont au moins l'un des principes actifs correspond à un ou plusieurs composés selon l'une quelconque des revendications 1 à 7, et qui contient également un support solide ou liquide et, le cas échéant, d'autres additifs.

**Revendications pour l'Etat contractant suivant : ES**

**1.** Procédé de préparation de composés représentés par la formule générale:

$$\begin{array}{l} Ar-X-B_1-N-B_2-Z-R \\ \qquad\qquad\quad | \\ \qquad\qquad\quad C=K \\ \qquad\qquad\quad | \\ \qquad\qquad\quad A \end{array} \qquad (I)$$

dans laquelle:

| | |
|---|---|
| Ar | représente un radical phényle; un radical phényle substitué par un ou plusieurs groupes sélectionnés parmi: atomes d'halogène, radical alkyle en $C_1$ à $C_4$, radical haloalkyle en $C_1$ à $C_4$, radical alkényle en $C_2$ à $C_4$, radical haloalkényle en $C_2$ à $C_4$, radical alcoxy en $C_1$ à $C_3$, ou radical haloalcoxy en $C_1$ à $C_4$; un radical pyridyle; et un radical pyridyle substitué par un ou plusieurs groupes sélectionnés parmi: atomes d'halogènes et radicaux haloalkyle en $C_1$ à $C_3$; |
| K, X et Z | représentent indépendamment les uns des autres, un atome d'oxygène ou un atome de soufre; |

| | |
|---|---|
| $B_1$ et $B_2$, | identiques ou différents l'un de l'autre, représentent des radicaux alkylène en $C_1$ à $C_6$, ramifiés ou linéaires; |
| R | représente un radical haloalkyle en $C_1$ à $C_6$ contenant entre 1 et 9 atomes d'halogène, un radical haloalkényle en $C_2$ à $C_8$ contenant entre 1 et 9 atomes d'halogène, un radical haloalcoxyalkyle en $C_3$ à $C_8$, un radical haloalcoxyalkényle en $C_3$ à $C_8$, l'atome d'halogène étant de préférence un atome de fluor; |
| A | représente un groupe sélectionné parmi: |

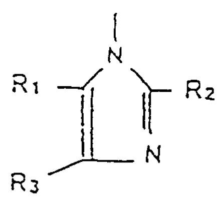 ; 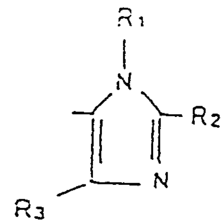 ;

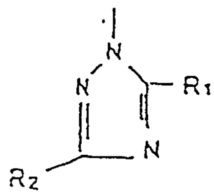 ; 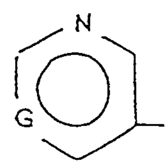 .

dans lesquelles:
$R_1$, $R_2$ et $R_3$ représentent H; et
G représente CH ou N;
ainsi que les sels et les complexes métalliques de ces composés;
et ledit procédé comprend les étapes suivantes:

a) la réaction des dérivés de formule générale (VII) et (VIII) selon la réaction:

$$\underset{(VII)}{Ar\text{-}X\text{-}B_1\text{-}Y} + \underset{(VIII)}{NH_2\text{-}B_2\text{-}Z\text{-}H} \longrightarrow \underset{(X)}{Ar\text{-}X\text{-}B_1\text{-}NH\text{-}B_2\text{-}Z\text{-}H}$$

dans un solvant organique et en présence d'une base à des températures de 0 °C à la température de reflux du solvant;

b) la réaction du composé de formule (X) qui résulte de l'étape (a) avec un dérivé de formule (XIII) selon la réaction:

$$(X) + \underset{(XVIII)}{CF_2{=}CX_1X_2} \longrightarrow \underset{(XIa)}{Ar\text{-}X\text{-}B_1\text{-}NH\text{-}B_2\text{-}Z\text{-}CF_2\text{-}CHX_1X_2}$$

dans des solvants alcooliques ou aprotiques dipolaires et en présence d'une base forte à des températures de -20 °C à 100 °C;

c) la réaction du composé de formule (XIa) résultant avec un dérivé carbonyle de A dans des solvants aromatiques ou halogénés à des températures de 0 °C à la température de reflux du solvant;

Y étant un atome d'halogène.

**2.** Procédé selon la revendication 1, caractérisé en ce que:

| | |
|---|---|
| Ar | représente un radical phényle, éventuellement substitué par 1 à 3 substituants sélectionnés parmi F, Cl et $CF_3$; et |
| K, X et Z | représentent un atome d'oxygène. |

**3.** Procédé selon l'une quelconque des revendications 1 et 2, caractérisés en ce que $B_1$ et $B_2$ représentent indépendamment l'un de l'autre, un radical éthylène ou un radical butylène ou propylène, linéaire ou ramifié.

**4.** Procédé selon l'une quelconque des revendications 1 à 3, caractérisés en ce que A est sélectionné parmi les radicaux 1-imidazolyle, 5-imidazolyle et 3-pyridyle.

**5.** Procédé selon la revendication 1, caractérisés en ce que les composés représentés par la formule (I) sont:

. N-[2-(1,1,2,2-tétrafluoroéthoxy)-éthyl]-N-[2-(3-trifluorométhylphénoxy)éthyl]-1-carboxyamido-imidazole,

. N-[2-(1,1,2,2-tétrafluoroéthoxy)-éthyl]-N-[2-(2,4,6-trichlorophénoxy)éthyl]-1-carboxyamido-imidazole,

. N-[2-(1,1,2,2-tétrafluoroéthoxy)-éthyl]-N-[2-(2,4,5-trichlorophénoxy)éthyl]-1-carboxyamido-imidazole,

. N-[2-(1,1,2,2-tétrafluoroéthoxy)-éthyl]-N-[2-(2,4-dichlorophénoxy)éthyl]-1-carboxyamido-imidazole,

. N-[2-(1,1,2,2-tétrafluoroéthoxy)-propyl]-N-[2-(2,4,6-trichlorophénoxy)éthyl]-1-carboxyamido-imidazole,

. N-[3-(1,1,2,2-tétrafluoroéthoxy)-propyl]-N-[2-(2,4,6-trichlorophénoxy)éthyl]-1-carboxyamido-imidazole,

. N-[2-(1,1,2-trifluoro-2-trifluorométhoxyéthoxy)-éthyl]-N-[2-(3-trifluorométhylphénoxy)éthyl]-1-carboxyamidoimidazole, et

. N-[2-(1,1,2,2-tétrafluoroéthoxy)-éthyl]-N-[2-(4-chlorophénoxy)-éthyl]-1-carboxyamido-pyridine.

**6.** Composition fongicide dont au moins l'un des principes actifs correspond à un ou plusieurs des composés produits selon l'une quelconque des revendications 1 à 5 et qui contient un support liquide ou solide et, le cas échéant, d'autres additifs.